# EUROPÄISCHE PATENTSCHRIFT

(11) **EP 1 613 217 B1**
(45) Veröffentlichungstag und Bekanntmachung des Hinweises auf die Patenterteilung: **04.06.2008**
(21) Anmeldenummer: 04722272.4
(22) Anmeldetag: 22.03.2004
(51) Int. Cl.: A61B 6/10

(54) **STRAHLENSCHUTZANORDNUNG MIT SEPARIERBARER UMHÜLLUNG**
RADIATION PROTECTION ARRANGEMENT COMPRISING A SEPARABLE COVER
DISPOSITIF DE PROTECTION ANTI-RADIATIONS COMPORTANT UNE ENVELOPPE SEPARABLE

(30) Priorität: 05.06.2003 DE 10325567
(43) Veröffentlichungstag der Anmeldung: 11.01.2006
(73) Patentinhaber: Mavig GmbH, 81829 München (DE)
(72) Erfinder: BALLSIEPER, Barbara, 82024 Taufkirchen (DE)
(74) Vertreter: Körfer, Thomas
(86) Internationale Anmeldenummer: PCT/EP2004/003027
(87) Internationale Veröffentlichungsnummer: WO 2004/107979

(56) Entgegenhaltungen:
- WO-A-02/064207
- DE-A- 3 338 122
- US-A- 4 196 355

## Beschreibung

Die vorliegende Erfindung betrifft eine Strahlenschutzanordnung, insbesondere eine Anordnung zur Abschirmung der durch eine Röntgenstrahlungsquelle abgestrahlten Röntgenstrahlung, welche beispielsweise zur Verwendung an einem angiographischen Arbeitsplatz vorgesehen ist.

Um die durch Röntgenuntersuchungen hervorgerufenen Strahlenbelastungen für die beteiligten Personen möglichst niedrig zu halten, ist es bereits seit langem bekannt, Strahlenschutzbekleidung zu verwenden. Eine beispielsweise aus der US 4,196,355 bekannte zweiteilige Strahlenschutzbekleidung besteht aus einer Weste zum Schutz des Oberkörpers sowie einem Rock zum Schutz des Unterkörpers, wobei die Weste und der Rock ein die Röntgenstrahlung abschirmendes Material aufweisen bzw. aus einem derartigen Material bestehen. Die für die Röntgenaufnahme zuständigen Personen, beispielsweise der Arzt oder ein Assistent tragen während der Untersuchungen die Weste und den Rock, um sich hierdurch gegenüber der Röntgenstrahlung zu schützen.

Die bekannten Strahlenschutzbekleidungen bieten zwar in bestimmten Fällen einen sehr effektiven Schutz gegen eine überhöhte Strahlenbelastung, an sogenannten angiographischen Arbeitsplätzen ist der damit erzielbare Schutz allerdings nur unzureichend. Die Strahlenexposition eines Untersuchers ist an einem derartigen Arbeitsplatz durch die multidirektionale Strahlenrichtung besonders hoch, so daß allein durch das Tragen einer Röntgenschutzbekleidung kein optimaler Strahlenschutz gewährleistet werden kann. Dementsprechend sind entsprechende Zusatzeinrichtungen zur Minimierung der Strahlenexposition erforderlich.

Die Minimierungspflicht der Strahlenschutzverordnung sieht dabei vor, daß die Dosisgrenzwerte nicht nur eingehalten, sondern möglichst auch noch unterschritten werden müssen. Es ist daher bekannt, sogenannte Unterkörperschutz-Anordnungen zu verwenden, welche seitlich an dem Patiententisch angeordnet werden. In einer einfachen Ausführung besteht ein derartiges Unterkörperschutz-System aus einer Abschirmmatte in Form einer in PCV eingeschichteten Bleigummimatte oder Bleifolie mit einem Bleigleichwert von 0,5 mm, die von der Tischebene bis zu dem Fußboden reicht und die von der Röntgenschutzkleidung des Untersuchers nicht bedeckten unteren Extremitäten vor Streustrahlung schützt. Dabei wird mit dem Bleigleichwert das Absorptionsverhalten eines Körpers, insbesondere eines Laminates beschrieben, das gegenüber Röntgenstrahlung eine Abschirmung aufweist, wie sie eine Bleiplatte der entsprechenden Dicke hat. Ein Material mit einem Bleigleichwert von 0,5 mm entspricht daher einer Bleiabschirmung mit einer Stärke von 0,5 mm.

In einer besonderen Ausführungsform besteht die oben beschriebene Unterkörperschutz-Anordnung aus mehreren PCV-Bleigummi-Lamellen, die seitlich nebeneinander und zumindest teilweise überlappend angeordnet sind. Darüber hinaus werden zur Optimierung des Strahlenschutzes sogenannte Strahlenschutzscheiben verwendet, die im oberen Bereich des Behandlungsplatzes angeordnet sind und den Kopf und Oberkörper des Untersuchers, insbesondere dessen Augen, Schilddrüse und Schultereckgelenk schützen. Auch an der Unterseite solcher Strahlenschutzscheiben können zusätzliche PVC-Bleigummimatten angeordnet werden, um die Abschirmung weiter zu verbessern.

Bei der Verwendung der oben beschriebenen Unterkörperschutz-Anordnungen ist zu berücksichtigen, daß diese Systeme während ihrer Verwendung häufig mit Körperflüssigkeiten, Kontrastmitteln oder anderen unsterilen Flüssigkeiten kontaminiert werden. Die Reinigung der Bleigummimatten oder der Bleigummi-Lamellen ist allerdings aufwendig und teuer, da hierbei die Bleifolien nicht beschädigt werden dürfen. Insbesondere die herkömmlichen Verfahren zum Sterilisieren durch Kochen oder mittels eines Autoklavs oder einer Bedampfung unter Schutzatmosphäre mit einem entsprechenden Gasgemisch sind nicht geeignet, da das Abschirmmaterial durch hohe Temperaturen beschädigt und damit die Schutzfunktion nicht mehr zuverlässig gewährleistet werden könnte.

Aus der US 5,417,225 A ist eine Strahlenschutzanordnung mit einer separierbaren Umhüllung bekannt. Dabei handelt es sich um eine Plastiktasche, die über die Schutzanordnung gezogen werden kann.

Eine weitere Problematik besteht darin, dass die für den Unterkörperschutz verwendeten Abschirmmatten eine feste Länge aufweisen, während hingegen der Behandlungstisch, an dem sie angeordnet und befestigt sind, in seiner Höhe in einem Bereich von ca. 70 cm bis ca. 120 cm verstellbar ist. Dies bedeutet, dass nur bei einer bestimmten Stellung des Behandlungstisches ein optimaler Schutz über die gesamte Höhe hinweg durch die Bleigummimatten gewährleistet ist. Befindet sich der Tisch jedoch in einer höheren Position, so sind die unteren Bereiche der Beine des Untersuchers nicht mehr geschützt. Befindet sich der Tisch in einer niedrigeren Position, so decken die Bleigummimatten zwar die gesamte Höhe des Tisches ab, das untere Ende der Matten liegt allerdings auf dem Boden auf, wobei sie in dieser Position besonders der Gefahr von Verunreinigungen ausgesetzt sind, da der Boden in der Umgebung des Behandlungs- oder Untersuchungstisches oftmals von Flüssigkeiten bedeckt ist. Weiterhin besteht die Gefahr der Trittbeschädigung.

Der vorliegenden Erfindung liegt dementsprechend die Aufgabe zugrunde, eine Möglichkeit anzugeben, Strahlenschutzanordnungen der zuvor beschriebenen Art auf möglichst einfache Weise rein und steril zu halten.

Die Aufgabe wird durch eine Strahlenschutzanordnung gemäß Anspruch 1 bzw. durch eine Umhüllung gemäß Anspruch 19 gelöst.

Die erfindungsgemäße Anordnung besteht im Wesentlichen aus zwei Elementen, zum einen aus einem Abschirmelement, welches aus dem Strahlenschutzmaterial besteht oder das Strahlenschutzmaterial enthält, und zum anderen aus einer das Abschirmelement vollständig umgebenden Umhüllung, welche über das Abschirmelement überziehbar und vollständig von dem Abschirmelement separierbar ist. Erfindungsgemäß weist die Umhüllung Befestigungselemente zur Befestigung an einer Trägerschiene auf.

Durch die Verwendung einer hinsichtlich ihrer Form an das Abschirmelement angepaßten aber dennoch separaten Umhüllung ist gewährleistet, daß das selbst nur aufwendig und teuer zu reinigende und sterilisierbare Abschirmelement in einer sterilen Umgebung - wie z.B. einem Operationssaal - verwendet werden kann, ohne daß es selbst nach jeder Benutzung intensiv gereinigt werden muß. Statt dessen ist es ausreichend, die abnehmbare Umhüllung zu entfernen und durch eine gereinigte Umhüllung zu ersetzen, was beispielsweise auch noch während der laufenden Untersuchung erfolgen kann. Die Umhüllung besteht dabei aus einem Material, welches wie herkömmliche Operationskittel in einer entsprechend geeigneten Vorrichtung schnell und standardmäßig sterilisiert werden kann. Für das Abschirmelement ist es hingegen ausreichend, dieses nur oberflächlich zu reinigen und mit einem Desinfektionsmittel abzureiben. Darüber hinaus stellt die Umhüllung auch einen gewissen Schutz dar, der das Abschirmelement vor einer unbeabsichtigten Beschädigung - beispielsweise mit einem Skalpell - schützt.

Gemäß einer besonders vorteilhaften Weiterbildung der vorliegenden Erfindung weist die Umhüllung Mittel auf, mit deren Hilfe die aus der Umhüllung und dem darin aufgenommenen Abschirmelement bestehende Anordnung längenverstellbar ist, was durch ein Umschlagen oder Abbinden der Umhüllung erfolgt. Hierdurch kann die Länge der Strahlenschutzanordnung individuell eingestellt und beispielsweise an die jeweilige Höhenstellung des Behandlungstisches angeglichen werden. Die zuvor beschriebene Problematik, daß die Anordnung zu kurz ist, um einen ausreichenden Schutz zu gewährleisten, oder zu lang ist und damit auf dem Boden aufliegt, wird hierdurch umgangen.

Ein Fixieren der Anordnung in der umgeschlagenen Form kann beispielsweise durch an der Umhüllung angeordnete Druckknöpfe, einen Klettverschluß oder einen Gurtverschluß erfolgen. Wesentlich dabei ist, daß die zur Fixierung der Anordnung erforderlichen Elemente ausschließlich an der Umhüllung angeordnet sind, nicht jedoch an dem Abschirmelement. Das Anbringen von Druckknöpfen oder anderen Verschlüssen an dem Abschirmelement ist nämlich nachteilig, da hierbei die zur Abschirmung der Röntgenstrahlung erforderliche Bleifolie beschädigt werden könnte.

Nachfolgend soll die Erfindung anhand der beiliegenden Zeichnung näher erläutert werden. Es zeigen:
- Fig. 1: einen angiographischen Arbeitsplatz, bei dem die Verwendung erfindungsgemäßer Strahlenschutzanordnungen vorgesehen ist;
- Fig. 2a: die Einzelteile eines ersten Ausführungsbeispieles einer erfindungsgemäßen Strahlenschutzanordnung;
- Fig. 2b: die in Fig. 2a dargestellte Anordnung im zusammengesetzten Zustand;
- Fig. 3: ein zweites Ausführungsbeispiel einer erfindungsgemäßen Strahlenschutzanordnung;
- Fig. 4: ein weiteres Ausführungsbeispiel einer Strahlenschutzanordnung, welche aus mehreren Lamellen besteht; und
- Fig. 5: ein viertes Ausführungsbeispiel einer Strahlenschutzanordnung zur Verwendung an einer Strahlenschutzscheibe.

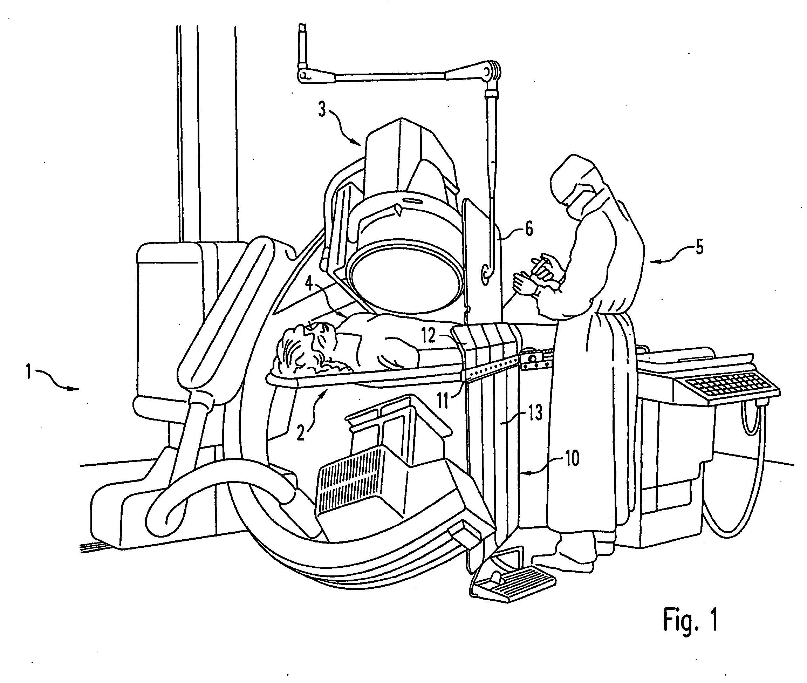

Fig. 1 zeigt einen allgemein mit dem Bezugszeichen 1 versehenen angiographischen Arbeitsplatz, dessen wesentliche Bestandteile ein höhenverstellbarer Patiententisch 2 sowie eine Röntgenanordnung 3 sind. Die Röntgenanordnung 3 ist schwenkbar gelagert, um eine möglichst flexible Ausrichtung des Röntgengenerators auf den Patienten 4 zu gewährleisten. Als Folge hiervon ergibt sich, daß Röntgenstrahlen und die entsprechenden Streustrahlen in verschiedensten Richtungen austreten können.

Um daher eine an dem Arbeitsplatz 1 tätige Person 5 möglichst umfangreich vor diesen Strahlungen schützen zu können, sind neben der Strahlenschutzbekleidung der Person 5 zusätzliche Strahlenschutzmaßnahmen vorgesehen. Im vorliegenden Fall bestehen diese aus einer Strahlenschutzscheibe 6, die eine Abschirmung des Oberkörpers des behandelnden Arztes 5 sowie dessen Kopfes ermöglichen soll. Darüber hinaus ist eine Unterkörperschutz-Anordnung 10 vorgesehen, welche am seitlichen Bereich des Behandlungstisches 2 befestigt ist. Diese Unterkörperschutz-Anordnung 10 besteht aus einem Oberteil 12, das an einer an dem Tisch 2 befestigten Trägerschiene 11 angeordnet ist, sowie aus mehreren an der Unterseite der Trägerschiene 11 befestigten Lamellen 13, die seitlich nebeneinander überlappend angeordnet sind. Die überlappende Anordnung der Lamellen 13 hat eine besonders hohe Flexibilität der Anordnung zur Folge, welche einen sehr wirksamen Strahlenschutz ermöglicht.

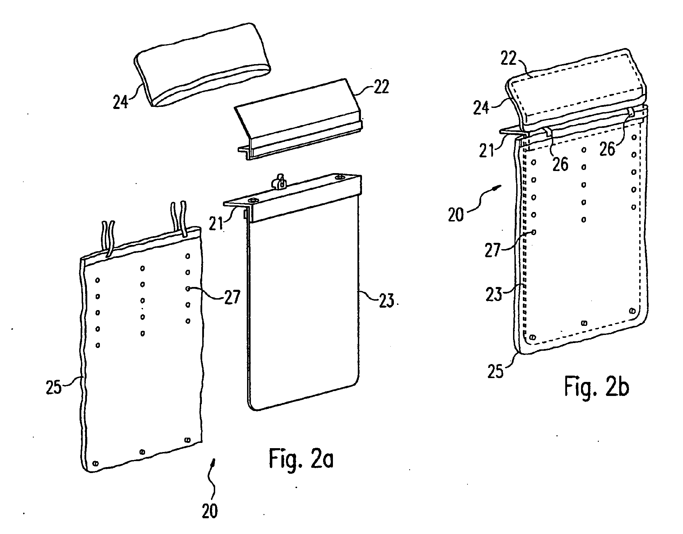

Um die aus in PVC eingelagerten Bleifolien bestehenden Strahlenschutz-Lamellen 13 nicht selbst reinigen oder sterilisieren zu müssen, ist erfindungsgemäß eine Umhüllung vorgesehen, welche zu deren Schutz über die Lamellen 13 übergezogen werden kann. Dies soll nachfolgend anhand eines ersten Ausführungsbeispiels in Fig. 2a erläutert werden.

Die in Fig. 2a in ihren Einzelteilen dargestellte Unterkörperschutz-Anordnung 20 besteht zunächst aus der bereits zuvor erwähnten Trägerschiene 21, an deren Oberseite ein Oberteil 22 zu befestigen ist. Zur Unterseite hin erstreckt sich eine Bleigummimatte 23. Das Oberteil 22 und die Bleigummimatte 23 weisen die zuvor erwähnten in PVC eingeschichteten Bleifolien auf und stellen jeweils ein Abschirmelement zur Abschirmung der Röntgenstrahlung dar. Die Bleifolien selbst weisen dabei zumindest einen Bleigleichwert von 0,5 mm auf, um eine ausreichende Abschirmung zu ermöglichen.

Das Oberteil 22 und die Bleigummimatte 23 sollen wie bereits erwähnt nicht oder nur selten gereinigt oder sterilisiert werden, da dies - ohne die Bleifolien zu beschädigen - nur mit einem sehr hohen Aufwand erfolgen kann. Aus diesem Grund sind jeweils Umhüllungen 24 und 25 vorgesehen, welche auf einfache Weise auf die beiden Abschirmelemente 22 und 23 aufgezogen und zur Reinigung und Sterilisation wieder abgenommen und entfernt werden können.

Die Umhüllung 24 für das Oberteil 22 besteht aus einem einfachen Futteral, welches auf das Oberteil 22 aufgestülpt wird. Auch die Umhüllung 25 für die untere Bleigummimatte 23 besteht aus einem in seinen Abmessungen etwa an die Größe der Bleigummimatte 23 angepaßten und zu einer Seite hin offenen Futteral, welches auf die Matte 23 aufgezogen und mittels Befestigungselementen 26 an der Trägerschiene 21 befestigt wird. Im dargestellten Beispiel werden die Befestigungselemente durch mehrere Schnüre 26 gebildet, mit denen die Umhüllung 25 an der Trägerschiene 21 festgebunden wird. Alternativ dazu könnte die Umhüllung 25 allerdings auch mittels eines Klettverschlusses oder mit Druckknöpfen an der Trägerschiene 21 befestigt werden.

Ein besonderes Merkmal der Umhüllung 25 für die untere Bleigummimatte 23 besteht in der Anordnung mehrerer Reihen von Druckknöpfen 27. Diese können dazu verwendet werden, die Umhüllung 25 mit der darin aufgenommenen Bleigummimatte 23 nach oben umzuschlagen und in dieser umgeschlagenen Position zu fixieren. Die Druckknöpfe 27 stellen somit eine Feststellvorrichtung dar, welche eine Längeneinstellung der aus der Bleigummimatte 23 und der Umhüllung 25 bestehenden Strahlenschutzanordnung ermöglicht. Hierdurch kann die Gesamtlänge an die Höhe des Behandlungstisches angepaßt werden, so daß zum einen über die gesamte Höhe hinweg eine Strahlenabschirmung erreicht wird, zum anderen allerdings verhindert wird, daß die Strahlenschutzanordnung auf dem Boden aufliegt und dort möglicherweise von Flüssigkeiten kontaminiert wird. Wesentlich ist, daß die Druckknöpfe 27 ausschließlich an der Umhüllung 25 angeordnet sind, nicht jedoch an der Bleigummimatte 23 selbst, da diese - insbesondere die Bleifolie - bei einem Anbringen von Knöpfen oder ähnlichen Elementen beschädigt werden würde.

Fig. 2a zeigt die Strahlenschutzanordnung 20 im zusammengesetzten Zustand. Wie der Darstellung entnommen werden kann, sind die Abschirmelemente 22 und 23 vollständig von den Umhüllungen 24 und 25 umgeben, so daß die Abschirmelemente 22 und 23 selbst nicht verschmutzt oder kontaminiert werden können. Die Umhüllungen 24 und 25, die vorzugsweise aus einem einfach zu reinigenden und zu sterilisierenden Stoff, beispielsweise dem allgemein in Operationssälen verwendeten grünen Baumwollstoff bestehen, können hingegen schnell entfernt und standardmäßig gereinigt werden. Das Auswechseln der Umhüllungen 24 und 25 kann dabei insbesondere auch noch während der Behandlung in kürzester Zeit erfolgen.

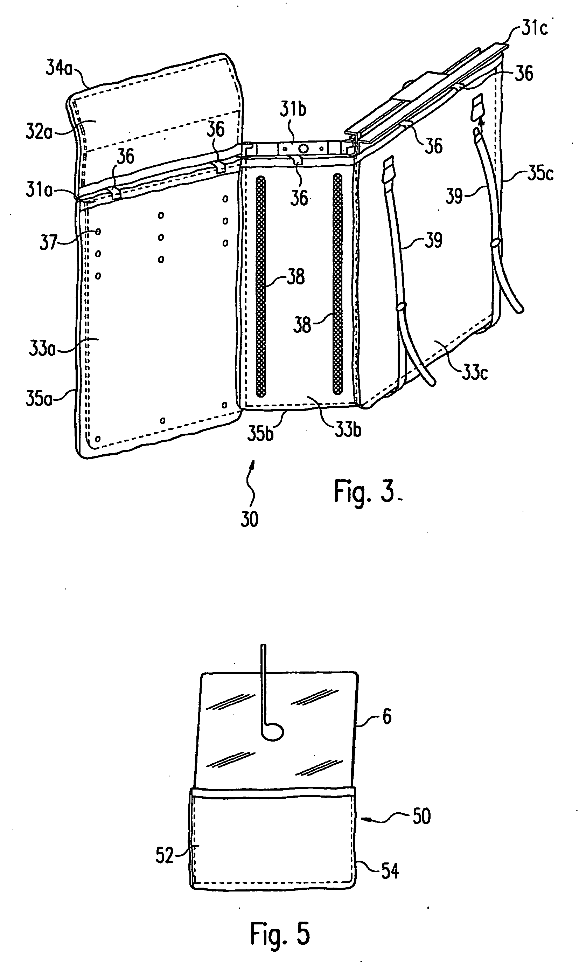

Fig. 3 zeigt ein weiteres Ausführungsbeispiel einer erfindungsgemäßen Strahlenschutzanordnung 30. Diese besteht im vorliegenden Fall aus drei einzelnen Elementen, die jeweils eine Trägerschiene 31a bis 31c aufweisen, an deren Unterseiten Bleigummimatten 33a bis 33c angeordnet sind. An der Oberseite der ersten Trägerschiene 31a ist ferner ein Strahlenschutzoberteil 32a mit einer entsprechenden Umhüllung 34a vorgesehen. Wiederum ist für jede einzelne Bleigummimatte 33a bis 33c eine eigene Umhüllung 35a bis 35c vorgesehen, welche hinsichtlich ihrer Breite und Länge etwa der Größe der entsprechenden Bleigummimatte 33a bis 33c entspricht. An den Oberseiten der Umhüllungen 35a bis 35c sind wieder Bänder 36 zum Befestigen an den Trägerschienen 31a bis 31c vorgesehen.

Alle drei Umhüllungen 35a bis 35c weisen die zuvor beschriebenen Feststellvorrichtungen auf, mit deren Hilfe die Länge der Strahlenschutzanordnung variiert werden kann. Die erste Umhüllung 35a weist - wie die bereits in Fig. 2a, 2b dargestellte. Umhüllung - Druckknöpfe 37 auf. Alternativ dazu ist allerdings auch die Verwendung eines Klettverschlusses möglich, wie dies bei der mittleren Anordnung dargestellt ist. Die Umhüllung 35b weist hierbei zwei sich über die gesamte Höhe hinweg erstreckende Klettbänder 38 auf, so daß bei einem Nachobenklappen des unteren Endes auf einfache Weise die Umhüllung 35b mit der Bleigummimatte 33b in der gewünschten Stellung fixiert werden kann. Eine dritte Möglichkeit zur Längeneinstellung besteht in der Verwendung von Gurten 39, wie dies bei der dritten Umhüllung 35c vorgesehen ist.

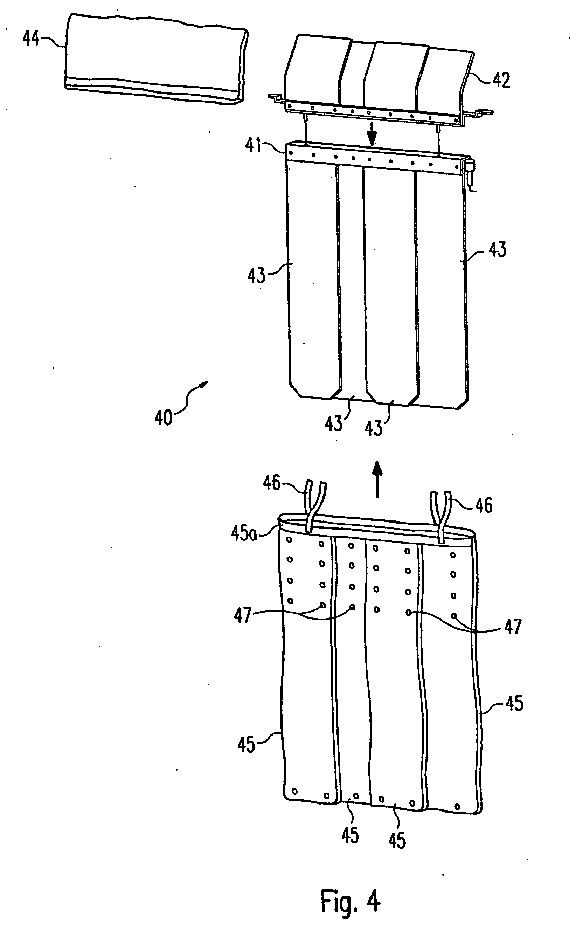

Ein besonders bevorzugtes Ausführungsbeispiel einer Unterkörperschutzanordnung ist in Fig. 4 dargestellt. Die hier dargestellte Strahlenschutzanordnung 40 zeichnet sich dadurch aus, daß an der Unterseite der Trägerschiene 41 nicht eine einzelne Bleigummimatte sondern statt dessen mehrere einzelne Bleigummi-Lamellen 43 seitlich nebeneinander aber überlappend angeordnet sind. Durch diese überlappende Anordnung der Lamellen 43 wird auf der einen Seite ein effektiver Strahlenschutz ermöglicht, auf der anderen Seite ist diese Anordnung besonders flexibel, so daß selbst in den unterschiedlichsten Situationen ein optimaler Strahlenschutz gewährleistet ist. Ferner ist wiederum ein Strahlenschutz-Oberteil 42 vorgesehen, welches eine einfache Umhüllung 44 aufweist.

Die Umhüllung für die einzelnen Lamellen 43 wird im vorliegenden Fall durch eine Anordnung gebildet, die aus mehreren seitlich nebeneinander angeordneten Futteralen 45 besteht, welche hinsichtlich ihrer Abmessungen an die Lamellen 43 angepaßt sind. Die Futterale 45 sind nur an ihren Oberseiten über einen gemeinsamen Bund 45a miteinander verbunden. An diesem Bund 45a sind wiederum die Befestigungsbänder 46 zur Befestigung der gesamten Umhüllung an der Montageleiste 41 vorgesehen.

Auch bei diesem bevorzugten Ausführungsbeispiel soll eine Höhenverstellbarkeit der Strahlenschutzanordnung ermöglicht werden, was wiederum durch die Verwendung von Druckknöpfen 47 erfolgt. Da jedes Futteral 45 eigene Druckknöpfe 47 aufweist, können die einzelnen Lamellen sogar individuell hinsichtlich ihrer Länge eingestellt werden. Selbstverständlich könnten allerdings auch - wie zuvor beschrieben - Klettverschlüsse oder Gurte zur Höhenverstellbarkeit verwendet werden.

Abschließend soll anhand von Fig. 5 ein weiteres Anwendungsbeispiel der erfindungsgemäßen Strahlenschutzanordnung erläutert werden. Hierbei soll ein Abschirmelement umhüllt werden, welches an der Unterseite einer Strahlenschutzscheibe 6, wie sie auch in Fig. 1 dargestellt ist, angeordnet ist. Die sich hierbei ergebende Gesamtanordnung ist in Fig. 5 dargestellt.

Ebenso wie bei den Unterkörperschutz-Systemen ist an der Unterkante der Strahlenschutzscheibe 6 ein Abschirmelement befestigt 52, welches eine zusätzliche Abschirmung der Röntgenstrahlung bewirkt. Dieses besteht wiederum aus einer eine Bleifolie aufweisenden PVC-Matte, welche von einer Umhüllung 54 eingefaßt ist. Die Umhüllung 54 entspricht in ihrer Ausführung im wesentlichen den für die Unterkörperschutz-Vorrichtungen vorgesehenen Umhüllungen. Ein Unterschied besteht allerdings darin, daß bei diesem Anwendungsfall eine Höhenverstellbarkeit der Strahlenschutzanordnung 50 nicht erforderlich ist und dementsprechend Feststellvorrichtungen in Form von Druckknöpfen, Klettverschlüssen oder Gurten nicht erforderlich sind. Auch hier ergibt sich allerdings der Vorteil, daß die Umhüllung 54 auf einfache Weise gereinigt und sterilisiert werden kann, ohne hierbei das empfindliche Abschirmelement 52 zu belasten. Ferner ist anzumerken, daß an der Unterseite der Strahlenschutzscheibe 6 auch eine Strahlenschutzanordnung entsprechend dem Ausführungsbeispiel in Fig. 4, also mit mehreren, überlappenden Lamellen angeordnet sein könnte, was insbesondere dann von Vorteil ist, wenn die Unterkante der Scheibe 6 keine Gerade ist sondern beispielsweise eine Krümmung aufweist.

Durch die vorliegende Erfindung wird somit ermöglicht, Strahlenschutzanordnungen auf einfache Weise rein und steril zu halten. Insbesondere bei Unterkörperschutz-Vorrichtungen wird darüber hinaus die Möglichkeit eröffnet, diese hinsichtlich ihrer Länge verstellbar zu gestalten und damit verschiedenen Situationen anzupassen.

## Patentansprüche

1. Strahlenschutzanordnung (20; 30; 40; 50) zur Abschirmung einer von einer Strahlungsquelle, insbesondere einer Röntgenquelle (3), abgestrahlten Strahlung, aufweisend
ein aus einem Strahlenschutzmaterial bestehendes bzw. Strahlenschutzmaterial aufweisendes Abschirmelement (22,23; 32a, 33a-c; 42,43; 52) sowie
eine hinsichtlich ihrer Form an das Abschirmelement (22, 23; 32a, 33a-c; 42, 43; 52) angepasste und dieses vollständig umgebende Umhüllung (24, 25; 34a, 35a-c; 44, 45; 54),
wobei die Umhüllung (24, 25; 34a, 35a-c; 44, 45; 54) über das Abschirmelement (22, 23; 32a, 33a-c; 42, 43; 52) überziehbar und vollständig von diesem separierbar ist,
wobei die Umhüllung ein hinsichtlich seiner Abmessungen an das Abschirmelement angepasstes und zu einer Seite hin offenes Futteral ist,
**dadurch gekennzeichnet,**
**dass** eine Befestigung der Umhüllung (24, 25; 34a, 35a-c; 44, 45; 54) mittels Befestigungselementen (26, 27, 37, 38, 39) an einer Trägerschiene (21; 31a, 31b, 31c; 41) vorgesehen ist

2. Strahlenschutzanordnung nach Anspruch 1,
**dadurch gekennzeichnet,**
**dass** die Umhüllung (24, 25; 34a, 35a-c; 44, 45; 54) aus einem mittels einer geeigneten Vorrichtung oder eines geeigneten Verfahrens sterilisierbaren Material besteht.

3. Strahienschutzanordnung nach Anspruch 1 oder 2,
**dadurch gekennzeichnet,**
**dass** die Umhüllung (24, 25; 34a, 35a-c; 44, 45; 54) mit dem darin angeordneten Abschirmelement (22, 23; 32a, 33a-c; 42, 43; 52) zur Längenveränderung in zumindest einer Richtung umschlagbar und mittels einer Feststellvorrichtung (27; 37, 38, 39; 47) in der umgeschlagenen Anordnung fixierbar ist

4. Strahlenschutzanordnung nach Anspruch 3,
**dadurch gekennzeichnet,**
**dass** die Feststellvorrichtung durch Druckknöpfe (27; 37; 47) gebildet ist.

5. Strahlenschutzanordnung nach Anspruch 3,
**dadurch gekennzeichnet,**
**dass** die Feststellvorrichtung durch einen Klettverschluss (38) gebildet ist.

6. Strahlenschutzanordnung nach Anspruch 3,
**dadurch gekennzeichnet,**
**dass** die Feststellvorrichtung durch einen Gurtverschluss (39) gebildet ist.

7. Strahlenschutzanordnung nach einem der vorherigen Ansprüche,
**dadurch gekennzeichnet,**
**dass** die Mittel zum Befestigen Bänder (26) sind.

8. Strahlenschutzanordnung nach einem der vorherigen Ansprüche,
**dadurch gekennzeichnet,**
**dass** die Mittel zum Befestigen der Umhüllung (24, 25; 34a, 35a-c; 44, 45; 54) Druckknöpfe sind.

9. Strahlenschutzanordnung nach einem der vorherigen Ansprüche,
**dadurch gekennzeichnet,**
**dass** die Mittel zum Befestigen der Umhüllung (24, 25; 34a, 35a-c; 44, 45; 54) Klettverschlüsse sind.

10. Strahlenschutzanordnung nach einem der vorherigen Ansprüche,
**dadurch gekennzeichnet,**
**dass** das Abschirmelement durch eine einzelne Matte (23, 33a-c, 52), welche ein die Röntgenstrahlung abschirmendes Material aufweist, und die Umhüllung durch ein hinsichtlich seiner Abmessungen an die Matte (23, 33a-c, 52) angepasstes und zu einer Seite hin offenes Futteral (25, 35a-c, 54) gebildet ist.

11. Strahlenschutzanordnung nach einem der Ansprüche 1 bis 9,
**dadurch gekennzeichnet,**
**dass** das Abschirmelement aus mehreren nebeneinander angeordneten Lamellen (43) besteht, welche ein die Röntgenstrahlung abschirmendes Material aufweisen und an einem Ende an einem gemeinsamen Trägerelement (41) befestigt sind,
wobei die Umhüllung mehrere längliche Futterale (45) zur Aufnahme jeweils einer Lamelle (43) aufweist, die an einem Ende über einen gemeinsamen Bund (45a) miteinander verbunden sind.

12. Strahlenschutzanordnung nach Anspruch 11,
**dadurch gekennzeichnet,**
**dass** die Lamellen (43) überlappend angeordnet sind.

13. Strahlenschutzanordnung nach einem der Ansprüche 3 bis 6 sowie Anspruch 11 oder 12,
**dadurch gekennzeichnet,**
**dass** jedes Futteral (45) eine eigene Feststellvorrichtung (47) zur Längenveränderung aufweist.

14. Strahlenschutzanordnung nach einem der vorherigen Ansprüche,
**dadurch gekennzeichnet,**
**dass** das Abschirmelement (22, 23; 32a, 33a-c; 42, 43; 52) eine von einer PVC-Hülle umgebene Bleifolie bzw. Bleigummimatte (23; 33a, 33b, 33c) aufweist.

15. Strahlenschutzanordnung nach Anspruch 14,
**dadurch gekennzeichnet,**
**dass** das Abschirmelement (22, 23; 32a, 33a-c; 42, 43; 52) einen Bleigleichwert von etwa 0,5 mm aufweist.

16. Strahlenschutzanordnung nach einem der vorherigen Ansprüche,
**dadurch gekennzeichnet,**
**dass** diese an der Unterseite einer Strahlenschutzscheibe (6) angeordnet ist.

17. Strahlenschutzanordnung nach einem der Ansprüche 1 bis 15,
**dadurch gekennzeichnet,**
**dass** diese einen seitlich an einem medizinischen Untersuchungs- oder Behandlungstisch (2) angeordneten Unterkörperschutz bildet.

18. Umhüllung (24, 25; 34a, 35a-c; 44, 45; 54) für ein aus einem Strahlenschutzmaterial bestehendes bzw. Strahlen-schutzmaterial aufweisendes Abschirmelement (22, 23; 32a, 33a-c; 42, 43; 52), welches zur Verwendung in einer Strahlenschutzanordnung (20; 30; 40; 50) zur Abschirmung einer von einer Strahlungsquelle, insbesondere einer Röntgenquelle (3), abgestrahlten Strahlung vorgesehen ist,
wobei die Umhüllung (24, 25; 34a, 35a-c; 44, 45; 54) derart ausgestaltet ist, dass sie über das Abschirmelement (22, 23; 32a, 33a-c; 42, 43; 52) überziehbar und vollständig von diesem wieder separierbar ist,
wobei die Umhüllung ein hinsichtlich seiner Abmessungen an das Abschirmelement (22, 23; 32a, 33a-c; 42, 43; 52) angepasstes und zu einer Seite hin offenes Futteral (25, 35a-c, 54) ist,
**dadurch gekennzeichnet,**
**dass** eine Befestigung der Umhüllung (24, 25; 34a, 35a-c; 44, 45; 54) mittels Befestigungselementen (26, 27, 37, 38, 39) an einer Trägerschiene (21; 31a, 31b, 31c; 41) vorgesehen ist.

19. Umhüllung nach Anspruch 18,
**dadurch gekennzeichnet,**
**dass** die Umhüllung (24, 25; 34a, 35a-c; 44, 45; 54) aus einem mittels einer geeigneten Vorrichtung oder eines geeigneten Verfahrens sterilisierbaren Material besteht.

20. Umhüllung nach Anspruch 18 oder 19,
**dadurch gekennzeichnet,**
**dass** die Umhüllung zur Längenveränderung in zumindest einer Richtung umschlagbar und mittels einer Feststellvorrichtung (27; 37, 38 ,39; 47) in der umgeschlagenen Anordnung fixierbar ist.

21. Umhüllung nach einem der Ansprüche 18 bis 20,
**dadurch gekennzeichnet,**
**dass** die Umhüllung mehrere längliche Futterale (45) aufweist, die an einem Ende über einen gemeinsamen Bund (45a) miteinander verbunden sind.

22. Umhüllung nach Anspruch 21,
**dadurch gekennzeichnet,**
**dass** jedes Futteral (45) eine eigene Feststellvorrichtung (47) zur Längenveränderung aufweist.

## Claims

1. A radiation protection arrangement (20; 30; 40; 50) for screening radiation emitted by a radiation source, in particular an X-ray source (3), having
a screening element (22, 23; 32a, 33a-c; 42, 43; 52) which comprises or includes a radiation protection material, and
a cover (24, 25; 34a, 35a-c; 44, 45; 54) which is matched in shape to the screening element (22, 23; 32a, 33a-c; 42, 43; 52) and completely surrounds the latter,
it being possible to pull the cover (24, 25; 34a, 35a-c; 44, 45; 54) over the screening element (22, 23; 32a, 33a-c; 42, 43; 52) and completely separate it therefrom,
the cover being a sheath which is matched in its dimensions to the screening element and is open to one side,
**characterised in**
**that** the cover (24, 25; 34a, 35a-c; 44, 45; 54) is provided with a means for securing it, by securing elements (26, 27, 37, 38, 39), to a carrier strip (21; 31a, 31b, 31c; 41).

2. A radiation protection arrangement according to Claim 1,
**characterised in**
**that** the cover (24, 25; 34a, 35a-c; 44, 45; 54) comprises a material which can be sterilised using a suitable device or a suitable process.

3. A radiation protection arrangement according to Claim 1 or 2,
**characterised in**
**that**, for the purpose of altering the length, the cover (24, 25; 34a, 35a-c; 44, 45; 54), with the screening element (22, 23; 32a, 33a-c; 42, 43; 52) arranged therein, can be turned up in at least one direction and fixed in the turned-up arrangement using a fixing device (27; 37, 38, 39; 47).

4. A radiation protection arrangement according to Claim 3,
**characterised in**
**that** the fixing device is formed by press studs (27; 37; 47) .

5. A radiation protection arrangement according to Claim 3,
**characterised in**
**that** the fixing device is formed by a hook-and-burr closure (38).

6. A radiation protection arrangement according to Claim 3,
**characterised in**
**that** the fixing device is formed by a tie closure (39).

7. A radiation protection arrangement according to one of the preceding claims,
**characterised in**
**that** the means for securing are tapes (26).

8. A radiation protection arrangement according to one of the preceding claims,
**characterised in**
**that** the means for securing the cover (24, 25; 34a, 35a-c; 44, 45; 54) are press studs.

9. A radiation protection arrangement according to one of the preceding claims,
**characterised in**
**that** the means for securing the cover (24, 25; 34a, 35a-c; 44, 45; 54) are hook-and-burr closures.

10. A radiation protection arrangement according to one of the preceding claims,
**characterised in**
**that** the screening element is formed by a single blanket (23, 33a-c, 52) which includes an X-ray screening material, and the cover is formed by a sheath (25, 35a-c, 54) which is matched in its dimensions to the blanket (23, 33a-c, 52) and is open to one side.

11. A radiation protection arrangement according to one of Claims 1 to 9,
**characterised in**
**that** the screening element comprises a plurality of slats (43) arranged next to one another and including an X-ray screening material and are secured at one end to a common carrier element (41), the cover having a plurality of elongate sheaths (45) for receiving a respective slat (43) and connected to one another at one end by way of a common cuff (45a).

12. A radiation protection arrangement according to Claim 11,
**characterised in**
**that** the slats (43) are arranged such that they overlap.

13. A radiation protection arrangement according to one of Claims 3 to 6 and Claim 11 or 12,
**characterised in**
**that** each sheath (45) has its own fixing device (47) for the purpose of altering the length.

14. A radiation protection arrangement according to one of preceding claims,
**characterised in**
**that** the screening element (22, 23; 32a, 33a-c; 42, 43; 52) includes a lead sheet or lead rubber blanket (23; 33a, 33b, 33c) surrounded by a PVC cover.

15. A radiation protection arrangement according to Claim 14,
**characterised in**
**that** the screening element (22, 23; 32a, 33a-c; 42, 43; 52) has a lead equivalence value of approximately 0.5 mm.

16. A radiation protection arrangement according to one of the preceding claims,
**characterised in**
**that** it is arranged on the underside of a radiation protection panel (6).

17. A radiation protection arrangement according to one of Claims 1 to 15,
**characterised in**
**that** it forms a lower body protection arranged to the side of a medical operating or treatment table (2).

18. A cover (24, 25; 34a, 35a-c; 44, 45; 54) for a screening element (22, 23; 32a, 33a-c; 42, 43; 52) which comprises or includes a radiation protection material and
is provided for use in a radiation protection arrangement (20; 30; 40; 50) for screening radiation emitted by a radiation source, in particular an X-ray source (3),
the cover (24, 25; 34a, 35a-c; 44, 45; 54) being constructed such that it can be pulled over the screening element (22, 23; 32a, 33a-c; 42, 43; 52) and completely separated therefrom again,
the cover being a sheath (25, 35a-c, 54) which is matched in its dimensions to the screening element (22, 23; 32a, 33a-c; 42, 43; 52) and is open to one side,
**characterised in**
**that** the cover (24, 25; 34a, 35a-c; 44, 45; 54) is provided with a means for securing it, by securing elements (26, 27, 37, 38, 39), to a carrier strip (21; 31a, 31b, 31c; 41).

19. A cover according to Claim 18,
**characterised in**
**that** the cover (24, 25; 34a, 35a-c; 44, 45; 54) comprises a material which can be sterilised using a suitable device or a suitable process.

20. A cover according to Claim 18 or 19,
**characterised in**
**that**, for the purpose of altering the length, the cover can be turned up in at least one direction and fixed in the turned-up arrangement using a fixing device (27; 37, 38, 39; 47).

21. A cover according to one of Claims 18 to 20,
**characterised in**
**that** the cover has a plurality of elongate sheaths (45) which are connected to one another at one end by way of a common cuff (45a).

22. A cover according to Claim 21,
**characterised in**
**that** each sheath (45) has its own fixing device (47) for the purpose of altering the length.

## Revendications

1. Dispositif de protection anti-radiations (20 ; 30 ; 40 ; 50) pour se protéger d'une radiation émise par une source de radiation, en particulier une source de rayons X (3) présentant
un élément de protection (22, 23 ; 32a, 33a-c ; 42, 43 ; 52) présentant un matériau de protection anti-radiations composé d'un matériau de protection anti-radiations et/ou présentant un matériau de protection ainsi que
une enveloppe (24, 25 ; 34a, 35a-c ; 44, 45 ; 54) adaptée quant à sa forme à l'élément de protection (22, 23 ; 32a, 33a-c ; 42, 43 ; 52) et entourant celui-ci complètement,
l'enveloppe (24, 25 ; 34a, 35a-c ; 44, 45 ; 54) pouvant être placée sur l'élément de protection (22, 23 ; 32a, 33a-c ; 42, 43 ; 52) et pouvant être complètement séparée de celui-ci, l'enveloppe étant un fourreau ouvert d'un côté et adapté quant à ses dimensions à l'élément de protection,
**caractérisé en ce que**
une fixation de l'enveloppe (24, 25 ; 34a, 35a-c ; 44, 45 ; 54) est prévue au moyen d'éléments de fixation (26, 27, 37, 38, 39) sur un rail porteur (21 ; 31a, 31b, 31c ; 41).

2. Dispositif de protection anti-radiations selon la revendication 1,
**caractérisé en ce**
**que** l'enveloppe (24, 25 ; 34a, 35a-c ; 44, 45 ; 54) se compose d'un matériau pouvant être stérilisé au moyen d'un dispositif approprié ou d'un procédé approprié.

3. Dispositif de protection anti-radiations selon la revendication 1 ou 2,
**caractérisé en ce**
**que** l'enveloppe (24, 25 ; 34a, 35a-c ; 44, 45 ; 54) peut être retournée avec l'élément de protection (22, 23 ; 32a, 33a-c ; 42, 43 ; 52) placé dedans pour une modification en longueur dans au moins une direction et peut être fixée au moyen d'un dispositif de fixation (27 ; 37, 38, 39 ; 47) dans le dispositif retourné.

4. Dispositif de protection anti-radiations selon la revendication 3,
**caractérisé en ce**
**que** le dispositif de fixation est constitué de boutons pressions (27 ; 37 ; 47).

5. Dispositif de protection anti-radiations selon la revendication 3,
**caractérisé en ce**
**que** le dispositif de fixation est constitué d'une fermeture Velcro (38).

6. Dispositif de protection anti-radiations selon la revendication 3,
**caractérisé en ce**
**que** le dispositif de fixation est constitué d'une fermeture à sangle (39).

7. Dispositif de protection anti-radiations selon l'une quelconque des revendications précédentes,
**caractérisé en ce**
**que** les moyens de fixation sont des bandes (26).

8. Dispositif de protection anti-radiations selon l'une quelconque des revendications précédentes,
**caractérisé en ce**
**que** les moyens pour fixer l'enveloppe (24, 25 ; 34a, 35a-c ; 44, 45 ; 54) sont des boutons pression.

9. Dispositif de protection anti-radiations selon l'une quelconque des revendications précédentes,
**caractérisé en ce**
**que** les moyens pour fixer l'enveloppe (24, 25 ; 34a, 35a-c ; 44, 45 ; 54) sont des fermetures Velcro.

10. Dispositif de protection anti-radiations selon l'une quelconque des revendications précédentes,
**caractérisé en ce**
**que** l'élément de protection est constitué d'un tapis individuel (23, 33a-c, 52), lequel présente un matériau protégeant de la radiation des rayons X et en ce que l'enveloppe est constituée d'un fourreau (25, 35a-c, 54) ouvert d'un côté, adapté quant à ses dimensions au tapis (23, 33a-c, 52).

11. Dispositif de protection anti-radiations selon l'une quelconque des revendications 1 à 9,
**caractérisé en ce**
**que** l'élément de protection se compose de plusieurs lamelles (43) juxtaposées, lesquelles présentent un matériau protégeant de la radiation des rayons X et qui, à une extrémité, sont fixées à un élément porteur commun (41), l'enveloppe présentant plusieurs fourreaux longitudinaux (45) pour loger respectivement une lamelle (43), qui sont reliés les uns aux autres à une extrémité par l'intermédiaire d'une attache commune (45a).

12. Dispositif de protection anti-radiations selon la revendication 11,
**caractérisé en ce**
**que** les lamelles (43) sont disposées en se chevauchant.

13. Dispositif de protection anti-radiations selon l'une quelconque des revendications 3 à 6 ainsi que 11 ou 12,
**caractérisé en ce**
**que** chaque fourreau (45) présente son propre dispositif de fixation (47) pour la modification en longueur.

14. Dispositif de protection anti-radiations selon l'une quelconque des revendications précédentes,
**caractérisé en ce**
**que** l'élément de protection (22, 23 ; 32a, 33a-c ; 42, 43 ; 52) présente un film en plomb et/ou un tapis protecteur en plomb (23 ; 33a, 33b, 33c) entouré d'une enveloppe en PVC.

15. Dispositif de protection anti-radiations selon la revendication 14,
**caractérisé en ce**
**que** l'élément de protection (22, 23 ; 32a, 33a-c ; 42, 43 ; 52) présente une épaisseur équivalente de plomb d'environ 0,5 mm.

16. Dispositif de protection anti-radiations selon l'une quelconque des revendications précédentes,
**caractérisé en ce**
**que** celui-ci est disposé sur la face inférieure d'une vitre de protection anti-radiations (6).

17. Dispositif de protection anti-radiations selon l'une quelconque des revendications 1 à 15,
**caractérisé en ce**
**que** celui-ci constitue une protection de la partie inférieure du corps disposée latéralement contre une table médicale d'auscultation ou de traitement (2).

18. Enveloppe (24, 25 ; 34a, 35a-c ; 44, 45 ; 54) pour un élément de protection (22, 23 ; 32a, 33a-c ; 42, 43 ; 52) se composant d'un matériau de protection anti-radiations et/ou présentant un matériau de protection anti-radiations, lequel est prévu pour l'utilisation dans un dispositif de protection anti-radiations (20 ; 30 ; 40 ; 50) pour se protéger d'une radiation émise par une source de radiation, en particulier une source de rayons X (3),
l'enveloppe (24, 25 ; 34a, 35a-c ; 44, 45 ; 54) étant conçue de façon à ce qu'elle puisse être placée sur l'élément de protection (22, 23 ; 32a, 33a-c ; 42, 43 ; 52) et qu'elle puisse être entièrement séparable de celui-ci,
l'enveloppe étant un fourreau (25, 34a-c, 54) ouvert d'un côté, adapté, quant à ses dimensions, à l'élément de protection (22, 23 ; 32a, 33a-c ; 42, 43 ; 52),
**caractérisée en ce**
**qu'**une fixation de l'enveloppe (24, 25 ; 34a, 35a-c ; 44, 45 ; 54) est prévue au moyen d'éléments de fixation (26, 27, 37, 38, 39) sur un rail porteur (21 ; 31a, 31b, 31c ; 41).

19. Enveloppe selon la revendication 18,
**caractérisée en ce**
**que** l'enveloppe (24, 25 ; 34a, 35a-c ; 44, 45 ; 54) se compose d'un matériau pouvant être stérilisé au moyen d'un dispositif approprié ou d'un procédé approprié.

20. Enveloppe selon la revendication 18 ou 19,
**caractérisée en ce**
**que** l'enveloppe peut être retournée pour la modification en longueur dans au moins une direction et peut être fixée au moyen d'un dispositif de fixation (27; 37; 38; 39; 47) dans le dispositif retourné.

21. Enveloppe selon l'une quelconque des revendications 18 à 20,
**caractérisée en ce**
**que** l'enveloppe présente plusieurs fourreaux longitudinaux (45), qui sont reliés les uns aux autres à une extrémité par l'intermédiaire d'une attache commune (45a).

22. Enveloppe selon la revendication 21,
**caractérisée en ce**
**que** chaque fourreau (45) présente son propre dispositif de fixation (47) pour la modification en longueur.
